# EUROPEAN PATENT SPECIFICATION

(11) **EP 2 846 160 B1**
(45) Date of publication and mention of the grant of the patent: **20.01.2016**
(21) Application number: 13183455.8
(22) Date of filing: 09.09.2013
(51) Int. Cl.: G01N 33/14, G01N 21/35, G01N 21/55, C12C 11/00

(54) **METHOD AND APPARATUS FOR BEER FERMENTATION**
VERFAHREN UND VORRICHTUNG ZUR BIERFERMENTATION
PROCÉDÉ ET APPAREIL POUR LA FERMENTATION DE LA BIÈRE

(43) Date of publication of application: 11.03.2015
(73) Proprietor: Alfa Laval Corporate AB, 221 00 Lund (SE)
(72) Inventor: Nordkvist, Mikkel, 3300 Frederiksværk (DK); Dorton, John Kyle, 1826 Frederiksberg (DK)
(74) Representative: Alfa Laval Attorneys

(56) References cited:
- WO-A1-00/17313
- WO-A1-2011/147958
- DE-A1- 1 935 818
- DE-A1- 3 807 621
- DE-A1- 3 920 397
- DE-A1- 3 927 856
- DE-A1- 19 547 473

## Description

### Technical Field

The invention relates to a method for beer fermentation, and to a beer fermentation apparatus. More specifically, the invention relates to increasing the efficiency of beer fermenters that operate by content agitation.

### Background

Modern brewing practice typically involves fermentation in large cylindroconical tanks, although other tank geometries are also used. Wort is produced in the brewhouse and transferred to the fermentation tank, while being chilled and aerated along the way. Also, yeast is normally added (pitched) to the wort in-line to the tank, which means that the yeast is added to the wort as the wort is led through a feeding pipe to the tank. The actual fermentation process involves conversion of fermentable extract (sugar) in the wort, by means of the yeast, into ethanol, CO₂ and a number of flavour compounds. During the process, vicinal diketones (VDK) are also formed from a precursor excreted by the yeast. To avoid producing beer with undesirable flavours, the VDK must be reduced by the yeast to sufficiently low levels, depending on beer type, before the tank content can be cooled and transferred to a storage vessel or be left in the tank until filtration. The majority of the yeast is removed from the fermenter either when a low extract concentration is reached, but prior to cooling (warm cropping), or after cooling to a specified temperature (cold cropping).

Traditionally, beer fermentation has relied on mixing by CO₂ bubble evolution and by natural convection generated by the cooling jackets that are used to remove heat of reaction. Commercial scale production in cylindroconical fermenters, however, shows that, without forced mixing, there is an initial phase before CO₂ generation commences where the yeast is heterogeneously distributed, with most of the yeast sediments settling into the cone after approximately 60% of the fermentable sugars have been consumed. Lately, a number of mixing systems based on recirculating part of the tank content and re-injecting it into the bulk liquid through a nozzle device has therefore been proposed for generating mixing, thereby distributing the yeast better and leading to a shorter fermentation time.

Still, monitoring the extract level, i.e. the amount of fermentable sugar in the wort, and VDK is based on manual sampling and measurement of these variables in a laboratory, and triggering of when to mix and when not to mix is typically controlled by a timed sequence while key events, like starting or stopping of cropping and starting of cooling, are triggered based on the results of laboratory measurements.

WO2007085880 describes an idea of optimizing a phenomenological model to represent the behaviour of a fermenter, which as such uses an approach based on fuzzy logic to update model parameters to match model predictions with plant data. Such fuzzy logic based approaches use trial and error processes that involve calibrating several parameters based on on-line measurements in a fermentation unit to automatically control some features; temperature, adjustment of agitator speed, etc. The idea is to store on-line measurements as well as laboratory analysis results in a computer connected to the plant control system, and parameter re-calibration is iteratively performed so as to reduce the mismatch between the plant data and the model calculation.

Other suggestions to use parameter measurements for controlling the fermentation process have been proposed in a number of patent documents.

DE3927856 describes generally how on-line measurements during fermentation may regulate e.g. agitation, heating and cooling.

US4959228 describes an on-line measurement of the specific gravity during fermentation in a mixing vessel.

US20030008340 describes how a biosensor is used for on-line measurements to potentially shorten beer fermentation time.

CN1733881 describes an on-line monitoring method for a beer fermentation process.

US7078201 describes how an oxidation reduction potential is monitored to decrease a fermentation time.

However, it is still believed that the fermentation processes suggested to date can be optimized, and that there is room for increased efficiency at the different process steps.

### Summary

It is an object of the invention to provide an improvement of the above described techniques and prior art. Thus, the invention relates to a method for obtaining automated beer fermentation. More specifically, some embodiments of the invention propose the use of on-line sensors, i.e. sensors connected to make measurements of the wort present in the vessel, that do not require that samples to be taken out of the fermentation vessel and to a lab for analysis. In addition, data retrieved by this sensor setup is used for automatic control of the fermentation process.

According to a first aspect, the invention is related to a method for beer fermentation, comprising the steps of:
- inserting wort and yeast into a vessel to initiate a fermentation process, the wort and yeast forming a vessel content;
- measuring, with an on-line measuring device, a first extract value that is representative of an extract level of the vessel content;
- automatically controlling a mixing device dependent on the first extract value, to withdraw content from the vessel and reinject it to the vessel for effecting mixing of the vessel content.

In one embodiment, the step of controlling the mixing device comprises the steps of:
- determining a first level of a process parameter of the fermentation process, at which to initiate cropping;
- determining a second level of the process parameter at which to cease the mixing;
- monitoring the process parameter;
- automatically shutting off the mixing device when the process parameter has reached the second level.

In one embodiment the process parameter is time and the first level is a future point in time determined by:
- measuring the first extract value at a first point in time and a second extract value that is representative of an extract level of the vessel content at a second point in time during the fermentation process;
- linearly extrapolating the first and second extract values at the two points in time, to the future point in time when the extract level would reach a predetermined extract level.

In one embodiment the second level of the process parameter is a future point in time preceding the first level of the process parameter, and is determined by:
- reducing the first level by a predetermined period of time.

In one embodiment the process parameter is extract, and the second level of the process parameter is a higher value of extract than the first level of extract.

In one embodiment the method comprises the step of:
- initiating cropping, when it is determined that the first level of the process parameter has been reached.

In one embodiment the method comprises the steps of:
- cropping by successively removing yeast from the vessel;
- measuring a level of yeast consistency of the removed yeast;
- automatically ceasing cropping when a predetermined yeast consistency has been reached.

In one embodiment the method comprises the steps of:
- sensing a fill level of the vessel;
- automatically setting the mixing device to a first flow level when a first fill level has been sensed during filling;
- automatically increasing to a second higher, flow level, when a second, higher, fill level has been sensed during filling.

In one embodiment the method comprises the steps of:
- measuring, with the on-line measuring device, a level of total diacetyl or total VDK of the vessel content;
- automatically initiate crash cooling of the vessel content when both
   i. a rate of change of the first extract value has fallen below a predetermined level, and
   ii. the level of total diacetyl or total VDK has fallen below a predetermined level.

In one embodiment the method comprises the step of:
- automatically setting the mixing device to a third flow level to assist diacetyl conversion.

In one embodiment the method comprises the step of:
- automatically setting the mixing device to a fourth flow level to assist cooling when crash cooling is initiated.

In one embodiment the on-line measuring device comprise a sensor connected to the vessel to sense attenuated total reflection by mid-infrared spectroscopy of the vessel content to obtain the first extract value.

According to a second aspect, the invention is related to an apparatus for beer fermentation, comprising a vessel for holding a vessel content comprising a mixture of at least wort and yeast during a fermentation process, an on-line measuring device including a sensor connected to the vessel to obtain a measurement of a first extract value that is representative of an extract level of the vessel content, a mixing device configured to withdraw content from the vessel and reinject it to the vessel for mixing of the vessel content, the on-line measuring device comprising a control device for automatically controlling at least the mixing device dependent on at least the first extract value.

In one embodiment the sensor is connected to the vessel to sense attenuated total reflection by mid-infrared spectroscopy of the vessel content to obtain the first extract value of the vessel content.

In one embodiment the control device comprises a data processing unit, and a non-transitory data memory holding control data for execution by the processing unit, whereby the control device is configured to control the apparatus to perform any one of the steps outlined in the method embodiments above.

Still other objectives, features, aspects and advantages of the invention will appear from the following detailed description as well as from the drawings.

### Drawings

Embodiments of the invention will now be described, by way of example, with reference to the accompanying schematic drawings, in which
Fig. 1 is a side view of a fermentation apparatus,
Fig. 2 shows a diagram of on-line measurement of real extract,
Fig. 3 shows a flowchart of a general representation of the invention,
Fig. 4 shows a flowchart of mixing control during vessel filling,
Fig. 5 shows a flowchart of mixing control before cropping,
Fig. 6 shows a flowchart of mixing control before and during cooling and
Fig. 7 shows a diagram of an on-line sensor fermentation curve with indicated mixing control events for a fermentation process.

### Detailed description

Generally speaking, the invention proposes automation of a beer fermentation process based on on-line sensor measurement of process parameter values for fermentation content. Embodiments relating to determination of real or apparent extract will be described in more detail. Extract level in a beer fermenter may be measured directly or may be inferred by a measurement of e.g. specific gravity, alcohol concentration, CO₂ gas volume leaving a fermenter, or any other time dependent variable that correlates with and is representative of real or apparent extract. The real extract is a measure of the sugars which are fermented and accounts for the density lowering effects of alcohol, and can be calculated from the initial and final densities. In one embodiment of the process real extract is measured directly using a method based on attenuated total reflection mid-infrared spectroscopy. This provides a clear benefit over the traditional laboratory use of hydrometers and the like, and allows for on-line measurement. Real extract normally falls in the range 0-25°P (Plato) during the course of fermentation.

Some embodiments may also employ on-line measurement of yeast concentration or consistency during yeast cropping, i.e. yeast removal. Whereas removed yeast initially may have a consistency in the range of 70-80%, it drops during cropping until a cut off value is reached.

On-line measurement may also be made of total diacetyl (diacetyl and its precursor) or total VDK concentration. Immediate precursors include, for example, a-acetolactate (AL). Typically, total VDK should be in the range of 50-200 ppb before cooling and subsequent processes can take place.

The on-line measurement is made for the purpose of operating a mixing device configured to mix the fermentation content. Preferably, the mixing device includes a jet mixing device, which works by withdrawing liquid by way of a pump from the vessel and reinjecting it into the bulk liquid via a nozzle device. The nozzle device, comprising one or more nozzles is positioned inside the vessel and may be based on either standard nozzles or may be of the venturi-type. Furthermore, the nozzles may be stationary. Alternatively, a rotating nozzle device is employed.

The linear velocity leaving said nozzle (calculated at the nozzle outlet and for a venturi-type based on the "outer" nozzle) may be in the range 5-40 m/s.

The proposed process also comprises a control device that, based on the on-line measurements, is capable of controlling the mixing device. Specifically, the control device is configured to adjust the linear velocity of a jet mixing device, and to turn the jet mixing device on and off based on measurements that relate to the level of extract, and possibly also based on measurements that relate to total VDK and yeast consistency. The control device may also be arranged to turn the mixing device off automatically to allow for cropping, and to start and stop a pump that is used for removal of the yeast crop. Pump start may be triggered by extract measurement in the case of warm cropping, and pump stop may be based on measurement of yeast consistency in a yeast crop line, which is a line configured for pulling of yeast from vessel.

In one embodiment the sensors to measure extract and total diacetyl/VDK are incorporated in a centralized measurement system in which samples are automatically withdrawn from individual fermenters in a network of fermenters and sent through a pipe in which the sensors are installed in-line, thereby needing fewer sensors than if the sensors are installed per tank.

Exemplary embodiments will now be described with reference to the drawings.

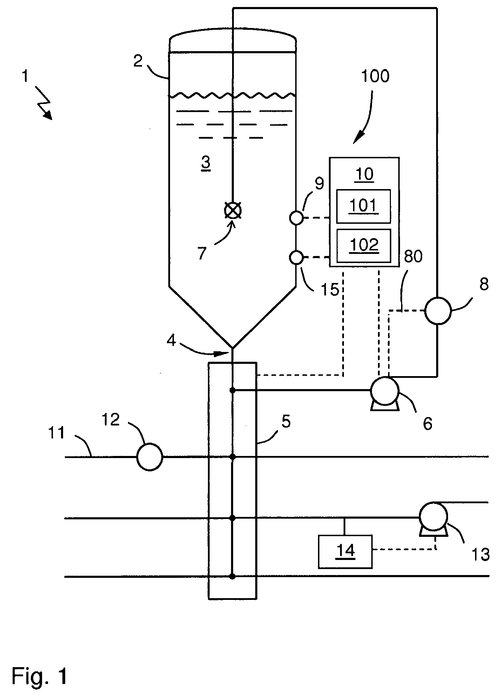

Fig. 1 schematically illustrates an apparatus 1 for beer fermentation. It should be noted that incorporated members of the fermenter apparatus 1 are illustrated merely based on functional purpose. The apparatus 1 comprises a vessel 2, preferably of a cylindroconical shape as illustrated. The vessel 2 is configured for holding a vessel content mixture 3 of at least wort and yeast during a fermentation process that takes place in the vessel 2. Normally, a fermentation plant has a number of vessels, or tanks, running in parallel, but the invention will be described for one vessel only for the sake of simplicity.

The vessel 2 has at least one port 4 arranged at its bottom, which by means of a valve system 5 connects the interior of the vessel 2 to one or more pipes 11 (conduits) for feeding or filling of the vessel 2. Also, the apparatus 1 incorporates a mixing device that is arranged to withdraw content from the vessel 2 and then reinject the content into the vessel 2, so as to provide agitation of the vessel content 3 by circulating the content 3. The mixing device includes a pump 6 connected to withdraw vessel content 3 from the vessel port 4, and a nozzle device 7 disposed inside the vessel 2, preferably at a fixed height over a bottom of the vessel 2. The mixing device 6, 7 may also include a flow meter 8 that is configured to measure a flow rate of fluid provided back into the vessel via the nozzle device 7. The nozzle device 7 may be a jet mixing device of any type as described above. The flow meter 8 may be arranged in a control loop 80 together with the pump 6. The pump 6 is also referred to as "mixer pump" 6.

An on-line measuring device 100 is incorporated to measure a first extract value of the vessel content 3, when the vessel content 3 is present in the vessel 2. The on-line measuring device 100 includes a sensor 9 that is connected to the vessel 2 and configured to sense a time-dependent variable of the fermentation process in the vessel 2. The time-dependent variable directly correlates with, and is thus representative of, the extract level of the vessel content 3. The sensor 9 is connected to a control device 10, which is part of the on-line measuring device 100 and which receives measurement signals from the sensor 9 and makes calculations to provide control signals for the fermentation process.

As explained above, extract sampling has historically been a cumbersome and to a high degree a manual process. Rather than extracting vessel content and testing it for its level of extract, it is hereby proposed to employ the sensor 9 in combination with the control device 10. The sensor 9 may be configured to measure e.g. alcohol concentration or CO₂ gas leaving the vessel 2, or any other variable that may be correlated to extract level of the vessel content 3.

In one embodiment the sensor 9 comprises an optical sensor that employs attenuated total reflection (ATR). Sensors that employ ATR include an optical crystal and make use of a property of total internal reflection in the crystal. Light is injected at one end of the crystal at a suitable angle to a front surface, and is then allowed to propagate by total internal reflection inside the crystal to an output end where light is collected. By placing the front surface in contact with the vessel content 3 to be tested, the amount and character of light passed to the detector will be affected, or attenuated. At the points of reflection in the interface between the front surface of the crystal and the vessel content 3, an evanescent wave is generated. The evanescent wave stretches at most a few µm into the vessel content 3, the exact value being determined by the wavelength of light, the angle of incidence and the indices of refraction for the ATR crystal and the vessel content 3. The resulting attenuation is hence dependent on the optical characteristics of the vessel content 3. Furthermore, by carrying out this process for different wavelengths, a characteristic profile for the sensed vessel content 3 is obtained. This is performed by Mid-Infrared (MIR) spectroscopy, which has been shown to provide very reliable sensing results when applied to a vessel content as described herein, i.e. a fermenter that comprises wort and yeast. The sensor 9 per se may be any suitable, conventional ATR and MIR based sensor.

It has been found that measurements made by means of an ATR MIR sensor 9 directly correlate with the level of extract.

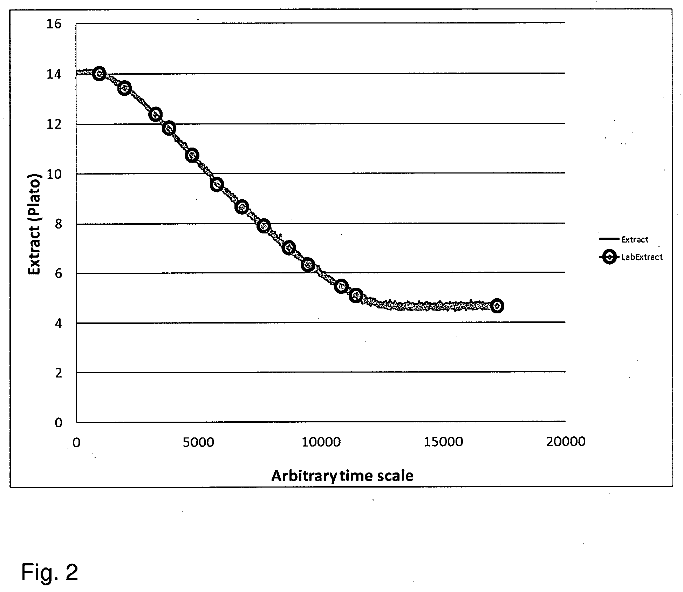

Fig. 2 illustrates on-line measurement of real extract using an ATR MIR sensor, as compared to laboratory measurements of the extract level. The shown example is measured on-line using the sensor 9 based on ATR MIR in a production scale fermenter, with a 5000 hL net volume. In accordance with one embodiment, the sensor 9 is mounted in the vessel 2 wall approximately half-way up from the port 4, through a flange connection (not shown).

Reverting to Fig. 1, the control device 10 receives measurement data from the sensor 9. The control device 10 is furthermore connected at least to the valve system 5 and to the mixer pump 6. The control device 10 comprises a data processing unit 101, such as a microprocessor, and a non-transitory data memory unit 102 holding control data for controlling at least the valve system 5 and the mixer pump 6. It should be understood that the control device 10 may thus incorporate a computer or PLC with associated computer program code stored in the memory unit 102. While the control device 10 is illustrated as being present beside the vessel 2, it may alternatively be located centrally in a brewery comprising several fermentation apparatuses like the apparatus 1, and be devised to receive measurements signals from plural sensors 9 and may likewise be devised to send out control signals to pumps and valves of plural different apparatuses. Measurement signal lines and control and signal lines, illustrated by dashed lines in Fig. 1, may be wired or alternatively wireless such as transmitted over e.g. radio.

One example of a process for fermenter control will now be described. It should be noted that not all process steps of beer fermentation are described in detail, since the general beer fermentation process as such is well known.

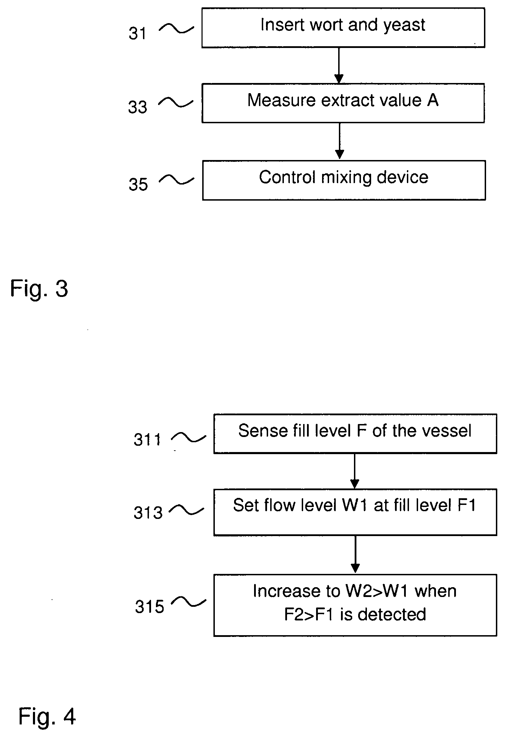

Referring to Fig. 3, filling of the vessel 2 is started at step 31, i.e. wort and yeast is inserted 31 into the vessel 2 to initiate a fermentation process. The wort and yeast form the vessel content 3. Filling may e.g. be performed through the conduit 11, via the valve system 5. A flow meter 12 (see Fig. 1) may be incorporated to measure a flow of content to the vessel 2. An alternative, or additional, means for measuring a fill level F of the vessel 2 may be provided by load cells (not shown) on which the vessel 2 is suspended.

In step 33, a first extract value A that is representative of an extract level E of the vessel content 3 is measured, by means of sensor 9, during the fermentation process. As mentioned, this measurement is preferably made with an ATR MIR sensor 9, on the content 3 present in the vessel 2.

In step 35, the mixing device is controlled based on the measurements, so as to provide mixing of the content 3 in the vessel 2.

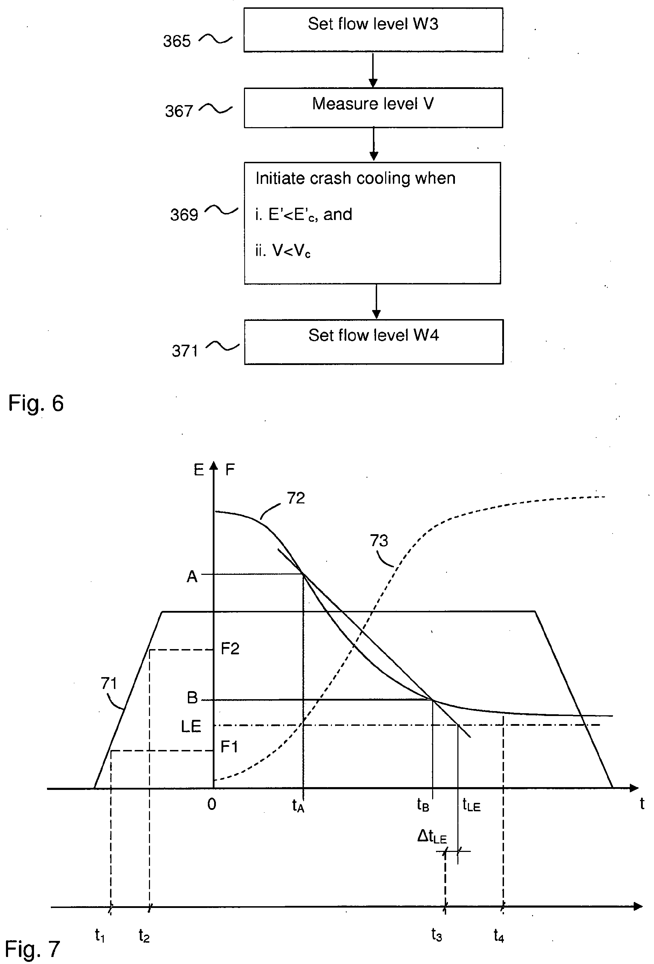

A more detailed description of the filling step 31 is explained with reference to Fig. 4, in accordance with one embodiment of the invention. The filling process may also be gathered from Fig. 7, which shows a diagram of various parameters during the fermentation process.

In step 311, a fill level F of the vessel is sensed. This may be carried out by integration using data from the flow meter 12, by load cells or by other known means. At the beginning of filling, the mixing device 6, 7 is turned off.

Step 313 indicates that when a first fill level F1 has been sensed during filling, the mixing device 6, 7 is automatically set to a first flow level W1. In one embodiment, the mixing device is started at a flow rate corresponding to a jet linear velocity of 5-20 m/s using the mixer pump 6, when the nozzle device 7 is covered by a volume of wort equaling a predetermined wort height, typically a height corresponding to 100-200 times the nozzle diameter. This corresponds to time point t₁ in Fig. 7, which occurs at the first fill level F1. The fill level F is indicated by curve 71.

In step 315, the flow level of the mixing device is then automatically increased to a second level W2, e.g. to jet linear velocity of typically 10-30 m/s, when a second, higher, fill level F2 has been sensed during filling. This corresponds to time point t₂ in Fig. 7, which occurs at fill level F2.

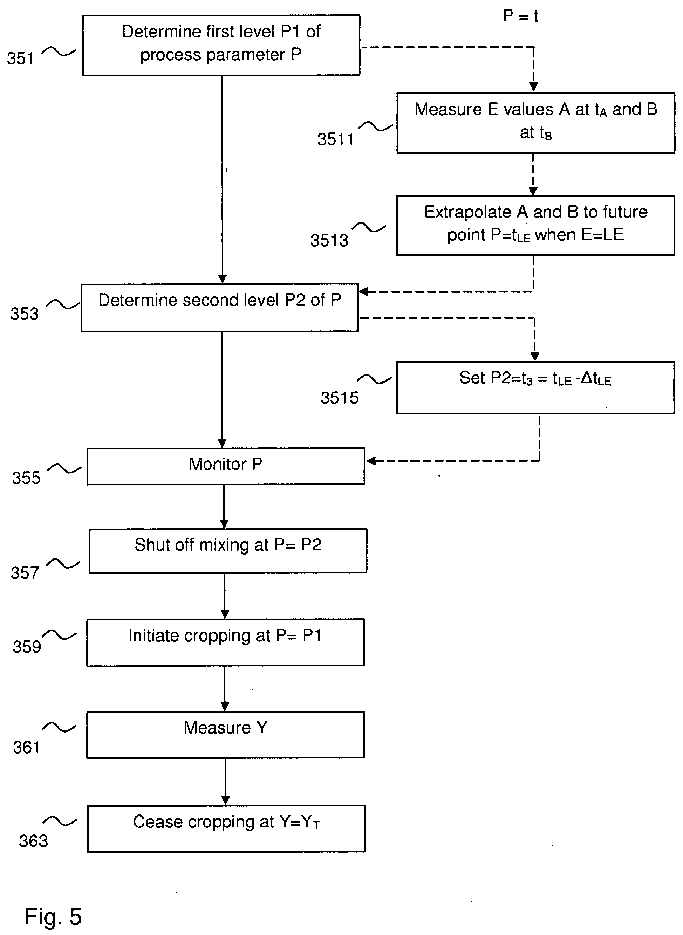

The step 35 of controlling the mixing device will now be described in more detail for an embodiment of the invention, with reference to Fig. 5, for a process that operates with cropping at "warm" conditions.

In step 351, a first level P1 of a process parameter P of the fermentation process, at which to initiate cropping, is determined. In one embodiment, the process parameter P is or correlates to extract E, and this step is carried out by selecting a predetermined extract level E=P1 at which cropping shall commence. An alternative embodiment will be described further below.

In step 353, a second level P2 of the process parameter P at which to cease mixing is determined. Ceasing of mixing shall typically be performed prior to cropping, and the second level P2 of the process parameter P thus represents a higher extract level E than the first level P1.

In step 355, the value of the process parameter P is monitored. This may e.g. be made by repetitive sensing using the aforementioned ATR MIR sensor 9, to determine a current extract level E.

In step 357, the mixing device is automatically shut off when the process parameter P has reached the second level P2.

In step 359, cropping is subsequently initiated, when it is determined that the first level P1 of the process parameter P has been reached. This step is preferably carried out by using a crop pump 13 (see Fig. 1), and involves successively removing yeast from the vessel 3.

In step 361, a level of yeast consistency Y of removed yeast is measured. Yeast consistency measurement is performed by a detector 14 (see Fig. 1), e.g. by measuring capacitance, turbidity or any other method from which yeast consistency may be inferred.

In step 363, cropping is automatically ceased, when a predetermined yeast consistency Y_{T} has been reached, which may depend on the type of beer to be brewed.

A variant of this embodiment will now be described, which also includes the boxes connected with dashed arrows at the right part of the flow chart of Fig. 5.

Reference will also be made to Fig. 7, in which the extract level E of the vessel content 3 is indicated by curve 72, and an alcohol signal is indicated by 73. In this embodiment the process parameter P is time t, and the first level P1 set in step 351 is a future point in time t_{LE}, rather than an extract level. This future point in time t_{LE}, which constitutes a target point in time for initiating cropping, is set using measurements from sensor 9.

In step 3511, the extract value E is sensed at two points in time during fermentation, by measuring the first extract value A at a first point in time t_{A}, and a second extract value B that is representative of an extract level E of the vessel content 3 at a second point in time t_{B}. The extract value E is preferably measured using sensor 9, to obtain corresponding extract levels.

In step 3513, a linear extrapolation is made of the corresponding extract level A, B at the two points in time t_{A}, t_{B}, to the future point in time t_{LE} when the extract would reach a limit extract level LE. Limit extract LE refers to the level to which the extract value asymptotically closes in, as can be gathered from Fig. 7, and represents the extract level that would have been obtained if no fermentable sugar would be left.

In step 3515, the second level P2 is also set as a future point in time t₃, by reducing the process parameter level t_{LE} by a predetermined period of time Δt_{LE}, typically in the range of 6-24 hours. In other words, the second level t₃ lies in the future when it is calculated, but it comes before the first level t_{LE}.

This embodiment has the benefit of requiring fewer measurements, and makes use of calculations by the control device 10 for establishing certain levels of a process parameter to monitor for controlling the mixing device. More specifically, by making these calculations on a limited number of measurements, the process parameter to monitor, with regard to the established levels, may simply be time t. Suitable points in time t_{A}, t_{B}, when to make the measurements to obtain extract level values are preferably selected so as to fall safely within the substantially linear slope part of the extraction level curve indicated in Fig. 2, so as to give reliable calculations of the time t_{LE.} Of course, measurements may be made at more than two points in time, so as to increase accuracy if needed.

In one embodiment, mixing of the vessel content 3 is performed also during diacetyl conversion, after cropping. This process is shown in Fig. 6.

In step 365, the mixing device is automatically set to a third flow level W3 to assist diacetyl conversion when cropping has ceased. In such an embodiment, the mixing device is started at a point in time t₄ as seen in Fig. 7, at a flow rate as measured by the flow meter 8, inferred by pressure measurements, or obtained during system calibration, corresponding to a jet linear velocity of typically 10-25 m/s. This step of automatically setting the mixing device to a third flow W3 level to assist diacetyl conversion may alternatively be carried out before cropping, in an embodiment employing cropping at cold conditions.

In step 367, a level V of total diacetyl or total VDK is measured for the vessel content 3. This step may be carried out by means of the on-line measuring device 100, including a suitable biosensor 15 at the vessel, connected to the control device 10.

In step 369, crash cooling of the vessel content 3 is subsequently automatically initiated when both the rate of change of extract level, E', has fallen below a predetermined level E'_{c}, e.g. E'_{c} =0.1-0.2 °P (degrees Plato) per day, and the level V of total diacetyl or total VDK has fallen below a predetermined level V_{c}, e.g. V_{c} =50-200 ppb. Cooling is continued until a desired terminal temperature has been reached. In one embodiment, mixing may also be performed during cooling, to speed up the crash cooling process. Crash cooling typically refers to cooling of the vessel content to allow any remaining yeast to sediment.

In step 371, the mixing device is automatically set to a fourth flow level W4 to assist cooling, e.g. to a rate corresponding to a jet linear velocity of typically 20-35 m/s. Preferably, the mixing device is stopped when a target temperature of the vessel content 3 has been reached if warm cropping has been performed and it is not desired to remove any further yeast prior to transfer from the vessel 2. Otherwise, the mixing during cooling should stop at a temperature which is higher than the target temperature, at about 6-24 hours prior to reaching the target temperature. If cropping is instead carried out at "cold" conditions, the mixing during cooling should cease at a temperature which is higher than the target temperature, at about 6-24 hours prior to reaching the target temperature, and the yeast should be removed using the crop pump 13 until a desired yeast consistency has been reached as measured by e.g. by capacitance, turbidity or other method by means of detector 14. Finally, the vessel 2 is emptied after a holding period of a predetermined time at the target temperature.

In the foregoing, the invention has been described with reference to a number of embodiments in various degree of detail. It should be noted that other related details of a beer fermentation process are well known to the skilled person, and that the foregoing description is sufficient for the skilled fermentation technician to carry out the invention. Also, the examples given are provided to show the best known mode of the invention, but that other embodiments falling under the scope of the claims are conceivable.

## Claims

1. A method for beer fermentation, comprising the steps of:
- inserting (31) wort and yeast into a vessel (2) to initiate a fermentation process, the wort and yeast forming a vessel content (3);
- measuring (32), with an on-line measuring device (100), a first extract value (A) that is representative of an extract level (E) of the vessel content (3);
- automatically controlling (35) a mixing device (6, 7) dependent on the first extract value (A), to withdraw vessel content (3) from the vessel (2) and reinject it into the vessel (2) for effecting mixing of the vessel content (3).

2. The method of claim 1, wherein the step (35) of controlling the mixing device (100) comprises the steps of:
- determining (351) a first level (P1) of a process parameter (P) of the fermentation process, at which to initiate cropping;
- determining (353) a second level (P2) of the process parameter at which to cease the mixing;
- monitoring (355) the process parameter (P);
- automatically shutting off (357) the mixing device (6, 7) when the process parameter (P) has reached the second level (P2).

3. The method of claim 2, wherein the process parameter (P) is time (t) and the first level (P1) is a future point in time (t_{LE}) determined by:
- measuring (3511) the first extract value (A) at a first point in time (t_{A}) and a second extract value (B) that is representative of an extract level (E) of the vessel content (3) at a second point in time (t_{B}) during the fermentation process;
- linearly extrapolating (3513) the first (A) and second (B) extract values at the two points in time (t_{A}, t_{B}), to the future point in time (T_{LE}) when the extract level would reach a predetermined extract level.

4. The method of claim 3, wherein the second level (P2) of the process parameter is a future point in time (t₃) preceding the first level (t_{LE}) of the process parameter, and is determined by:
- reducing (3515) the first level (t_{LE}) by a predetermined period of time (Δt_{LE}).

5. The method of claim 2, wherein the process parameter (P) is extract (E), and the second level (P2) of the process parameter is a higher value of extract than the first level (P1) of extract.

6. The method of any of the preceding claims 2-5, comprising the step of:
- initiating cropping (359), when it is determined that the first level (t_{LE}) of the process parameter has been reached.

7. The method of any of the preceding claims, comprising the steps of:
- cropping (359) by successively removing yeast from the vessel (3);
- measuring (361) a level of yeast consistency (Y) of the removed yeast;
- automatically ceasing (363) cropping when a predetermined yeast consistency (Y_{T}) has been reached.

8. The method of any of the preceding claims, comprising the steps of:
- sensing (311) a fill level of the vessel (2);
- automatically setting (313) the mixing device (6, 7) to a first flow level (W1) when a first fill level (F1) has been sensed during filling;
- automatically increasing (315) to a second higher, flow level (W2), when a second, higher, fill level (F2) has been sensed during filling.

9. The method of claim 8, comprising the steps of:
- measuring (367), with the on-line measuring device (100), a level (V) of total diacetyl or total VDK of the vessel content (3);
- automatically initiate (369) crash cooling of the vessel content (3) when both
i. a rate of change of the first extract value (A) has fallen below a predetermined level, and
ii. the level of total diacetyl or total VDK has fallen below a predetermined level.

10. The method of claim 9, comprising the step of:
- automatically setting (365) the mixing device (6, 7) to a third flow level (W3) to assist diacetyl conversion.

11. The method of claim 10, comprising the step of:
- automatically setting (371) the mixing device (6, 7) to a fourth flow level (W4) to assist cooling when crash cooling is initiated.

12. The method of any of the preceding claims, wherein the on-line measuring device (100) comprise a sensor (9) connected to the vessel (2) to sense attenuated total reflection by mid-infrared spectroscopy of the vessel content (3) to obtain the first extract value (A).

13. An apparatus (1) for beer fermentation, comprising a vessel (2) for holding a vessel content (3) comprising a mixture of at least wort and yeast during a fermentation process, an on-line measuring device (100) including a sensor (9) connected to the vessel (2) to obtain a measurement of a first extract value (A) that is representative of an extract level of the vessel content (3), a mixing device (6, 7) configured to withdraw content from the vessel (2) and reinject it to the vessel (2) for mixing of the vessel content (3), the on-line measuring device (100) comprising a control device (10) for automatically controlling at least the mixing device (6, 7) dependent on at least the first extract value (A).

14. The apparatus of claim 13, wherein the sensor (9) is connected to the vessel (2) to sense attenuated total reflection by mid-infrared spectroscopy of the vessel content (3) to obtain the first extract value (A) of the vessel content.

15. The apparatus of claim 13 or 14, wherein the control device (10) comprises a data processing unit (101), and a non-transitory data memory (102) holding control data for execution by the processing unit (101), whereby the control device (10) is configured to control the apparatus to perform any one of the steps outlined in the preceding method claims 1 - 12.

## Patentansprüche

1. Verfahren zur Biergärung, die Schritte umfassend:
- Einleiten (31) von Würze und Hefe in einen Gärtank (2), um einen Gärprozess einzuleiten, wobei die Würze und die Hefe einen Gärtankinhalt (3) bilden;
- mit einem Online-Messgerät (100) erfolgendes Messen (32) eines ersten Extraktionswerts (A), der ein Extraktionsmaß (E) des Gärtankinhalts (3) darstellt;
- automatisches Steuern (35) eines Mischgeräts (6, 7) in Abhängigkeit von dem ersten Extraktionswert (A), um Gärtankinhalt (3) aus dem Gärtank (2) abzuleiten und ihn zum effektiven Mischen des Gärtankinhalts (3) erneut in den Gärtank (2) einzuleiten.

2. Verfahren nach Anspruch 1, worin der Schritt (35) des Steuerns des Mischgeräts (100) die Schritte umfasst:
- Bestimmen (351) eines ersten Maßes (P1) eines Prozessparameters (P) des Gärprozesses, bei dem das Sammeln einzuleiten ist;
- Bestimmen (353) eines zweiten Maßes (P2) des Prozessparameters (P), bei dem das Mischen zu beenden ist;
- Überwachen (355) des Prozessparameters (P);
- automatisches Abschalten (357) des Mischgeräts (6, 7), wenn der Prozessparameter (P) das zweite Maß (P2) erreicht hat.

3. Verfahren nach Anspruch 2, worin der Prozessparameter (P) die Zeit (t) ist und das erste Maß (P1) ein zukünftiger Zeitpunkt (t_{LE}) ist, der bestimmt wird durch:
- Messen (3511) des ersten Extraktionswerts (A) bei einem ersten Zeitpunkt (t_{A}) und eines zweiten Extraktionswerts (B), der ein Extraktionsmaß (E) des Gärtankinhalts (3) bei einem zweiten Zeitpunkt (t_{B}) während des Gärprozesses darstellt;
- lineares Extrapolieren (3513) der ersten (A) und zweiten (B) Extraktionswerte bei den beiden Zeitpunkten (t_{A}, t_{B}) zu dem zukünftigen Zeitpunkt (t_{LE}), bei dem das Extraktionsmaß ein vorbestimmtes Extraktionsmaß erreichen würde.

4. Verfahren nach Anspruch 3, worin das zweite Maß (P2) des Prozessparameters (P) ein zukünftiger Zeitpunkt (t₃) ist, der dem ersten Maß (t_{LE}) des Prozessparameters vorausgeht und bestimmt wird durch:
- Vermindern (3515) des ersten Maßes (t_{LE}) um einen vorbestimmten Zeitabschnitt (Δt_{LE}).

5. Verfahren nach Anspruch 2, worin der Prozessparameter (P) die Extraktion (E) ist und das zweite Maß (P2) des Prozessparameters ein höherer Wert der Extraktion als das erste Maß (P1) der Extraktion ist.

6. Verfahren nach Anspruch 2 bis 5, den Schritt umfassend:
- Einleiten des Sammelns (359), wenn bestimmt wird, dass das erste Maß (t_{LE}) des Prozessparameters erreicht worden ist.

7. Verfahren nach einem der vorhergehenden Ansprüche, die Schritte umfassend:
- Sammeln (359) durch fortlaufendes Entnehmen von Hefe aus dem Gärtank (3);
- Messen (361) eines Maßes der Hefekonsistenz (Y) der entnommenen Hefe;
- automatisches Beenden (363) des Sammelns, wenn eine vorbestimmte Hefekonsistenz (Y_{T}) erreicht worden ist.

8. Verfahren nach einem der vorhergehenden Ansprüche, die Schritte umfassend:
- Erfassen (311) eines Füllstandes des Gärtanks (2);
- automatisches Einstellen (313) des Mischgeräts (6, 7) auf ein erstes Strömungsmaß (W1), wenn während des Füllens ein erster Füllstand (F1) erfasst worden ist;
- automatisches Steigern (315) auf ein zweites, höheres Strömungsmaß (W2), wenn während des Füllens ein zweiter, höherer Füllstand (F2) erfasst worden ist.

9. Verfahren nach Anspruch 8, die Schritte umfassend:
- mit dem Online-Messgerät (100) erfolgendes Messen (367) eines Maßes (V) an Gesamt-Diacetyl oder Gesamt-VDK des Gärtankinhalts (3);
- automatisches Einleiten (369) der Schnellkühlung des Gärtankinhalts (3), wenn sowohl
i. eine Änderungsgeschwindigkeit des ersten Extraktionswerts (A) unter ein vorbestimmtes Maß gesunken ist als auch
ii. das Maß an Gesamt-Diacetyl oder Gesamt-VDK unter ein vorbestimmtes Maß gesunken ist.

10. Verfahren nach Anspruch 9, den Schritt umfassend:
- automatisches Einstellen (365) des Mischgeräts (6, 7) auf ein drittes Strömungsmaß (W3), um die Diacetyl-Umwandlung zu unterstützen.

11. Verfahren nach Anspruch 10, den Schritt umfassend:
- automatisches Einstellen (371) des Mischgeräts (6, 7) auf ein viertes Strömungsmaß (W4), um die Schnellkühlung zu unterstützen, wenn die Schnellkühlung eingeleitet wird.

12. Verfahren nach einem der vorhergehenden Ansprüche, worin das Online-Messgerät (100) einen Sensor (9) umfasst, der mit dem Gärtank (2) verbunden ist, um die gedämpfte Totalreflexion durch Mittelinfrarot-Spektroskopie des Gärtankinhalts (3) zu erfassen, um den ersten Extraktionswert (A) zu erlangen.

13. Vorrichtung (1) zur Biergärung, umfassend: einen Gärtank (2) zum Aufnehmen eines Gärtankinhalts (3), welcher eine Mischung von mindestens Würze und Hefe umfasst, während eines Gärprozesses, ein Online-Messgerät (100), das einen Sensor (9) aufweist, der mit dem Gärtank (2) verbunden ist, um einen Messwert eines ersten Extraktionswerts (A) zu erlangen, der ein Extraktionsmaß des Gärtankinhalts (3) darstellt, ein Mischgerät (6, 7), das dafür konfiguriert ist, den Inhalt aus dem Gärtank (2) abzuleiten und ihn zum Mischen des Gärtankinhalts (3) erneut in den Gärtank (2) einzuleiten, wobei das Online-Messgerät (100) ein Steuerungsgerät (10) zum automatischen Steuern zumindest des Mischgeräts (6, 7) in Abhängigkeit von zumindest dem ersten Extraktionswert (A) umfasst.

14. Vorrichtung nach Anspruch 13, worin der Sensor (9) mit dem Gärtank (2) verbunden ist, um die gedämpfte Totalreflexion durch Mittelinfrarot-Spektroskopie des Gärtankinhalts (3) zu erfassen, um den ersten Extraktionswert (A) des Gärtankinhalts zu erlangen.

15. Vorrichtung nach Anspruch 13 oder 14, worin das Steuerungsgerät (10) eine Datenverarbeitungseinheit (101) und einen nichtflüchtigen Datenspeicher (102), der Steuerungsdaten zur Ausführung durch die Datenverarbeitungseinheit (101) aufnimmt, umfasst, wobei das Steuerungsgerät (10) dafür konfiguriert ist, die Vorrichtung zu steuern, um einen der in den vorhergehenden Verfahrensansprüchen 1 bis 12 skizzierten Schritte durchzuführen.

## Revendications

1. Procédé pour la fermentation de la bière, comprenant les étapes suivantes :
- l'introduction (31) de moût et de levure dans une cuve (2) pour commencer un processus de fermentation, le moût et la levure formant un contenu de cuve (3) ;
- la mesure (32), à l'aide d'un dispositif de mesure en ligne (100), d'une première valeur d'extrait (A) qui est représentative d'un niveau d'extrait (E) du contenu de cuve (3) ;
- la commande automatique (35) d'un dispositif de mélange (6, 7) en fonction de la première valeur d'extrait (A), pour retirer le contenu de cuve (3) de la cuve (2) et le réinjecter dans la cuve (2) pour effectuer le mélange du contenu de cuve (3).

2. Procédé selon la revendication 1, dans lequel l'étape (35) de commande du dispositif de mélange (100) comprend les étapes suivantes :
- la détermination (351) d'un premier niveau (P1) d'un paramètre de processus (P) du processus de fermentation, où l'on doit commencer la récolte ;
- la détermination (353) d'un second niveau (P2) du paramètre de processus où l'on doit cesser le mélange ;
- la surveillance (355) du paramètre de processus (P) ;
- l'arrêt automatique (357) du dispositif de mélange (6, 7) lorsque le paramètre de processus (P) a atteint le second niveau (P2).

3. Procédé selon la revendication 2, dans lequel le paramètre de processus (P) est le temps (t) et le premier niveau (P1) est un moment donné futur (t_{LE}) déterminé par :
- la mesure (3511) de la première valeur d'extrait (A) à un premier moment donné (t_{A}) et d'une seconde valeur d'extrait (B) qui est représentative d'un niveau d'extrait (E) du contenu de cuve (3) à un second moment donné (t_{B}) au cours du processus de fermentation ;
- l'extrapolation linéaire (3513) des première (A) et seconde (B) valeurs d'extrait aux deux moments donnés (t_{A}, t_{B}), sur le moment donné futur (t_{LE}) où le niveau d'extrait doit atteindre un niveau d'extrait prédéterminé.

4. Procédé selon la revendication 3, dans lequel le second niveau (P2) du paramètre de processus est un moment donné futur (t₃) précédent le premier niveau (t_{LE}) du paramètre de processus, et est déterminé par :
- la réduction (3515) du premier niveau (t_{LE}) d'une période de temps prédéterminée (Δt_{LE}).

5. Procédé selon la revendication 2, dans lequel le paramètre de processus (P) est l'extrait (E), et le second niveau (P2) du paramètre de processus est une valeur d'extrait supérieure au premier niveau (P1) d'extrait.

6. Procédé selon l'une quelconque des revendications précédentes 2 à 5, comprenant l'étape suivante :
- le démarrage de la récolte (359), lorsqu'on a déterminé que le premier niveau (t_{LE}) du paramètre de processus a été atteint.

7. Procédé selon l'une quelconque des revendications précédentes, comprenant les étapes suivantes :
- la récolte (359) en retirant successivement la levure de la cuve (3) ;
- la mesure (361) d'un niveau de consistance de la levure (Y) de la levure retirée ;
- l'arrêt automatique (363) de la récolte lorsqu'une consistance de levure prédéterminée (Y_{T}) a été atteinte.

8. Procédé selon l'une quelconque des revendications précédentes, comprenant les étapes suivantes :
- la détection (311) d'un niveau de remplissage de la cuve (2) ;
- le réglage automatique (313) du dispositif de mélange (6, 7) à un premier niveau d'écoulement (W1) lorsqu'un premier niveau de remplissage (F1) a été détecté au cours du remplissage ;
- l'augmentation automatique (315) à un deuxième niveau d'écoulement (W2) plus élevé, lorsqu'un second niveau de remplissage (F2) plus élevé a été détecté au cours du remplissage.

9. Procédé selon la revendication 8, comprenant les étapes suivantes :
- la mesure (367), à l'aide du dispositif de mesure en ligne (100), d'un niveau (V) de diacétyle total ou de VDK total du contenu de cuve (3) ;
- le démarrage automatique (369) du refroidissement rapide du contenu de cuve (3) lorsqu'à la fois
i. un taux de variation de la première valeur d'extrait (A) est tombé en dessous d'un niveau prédéterminé, et
ii. le niveau de diacétyle total ou de VDK total est tombé en dessous d'un niveau prédéterminé.

10. Procédé selon la revendication 9, comprenant l'étape suivante :
- le réglage automatique (365) du dispositif de mélange (6, 7) à un troisième niveau d'écoulement (W3) pour faciliter la transformation de diacétyle.

11. Procédé selon la revendication 10, comprenant l'étape suivante :
- le réglage automatique (371) du dispositif de mélange (6, 7) à un quatrième niveau d'écoulement (W4) pour faciliter le refroidissement lorsque le refroidissement rapide a commencé.

12. Procédé selon l'une quelconque des revendications précédentes, dans lequel le dispositif de mesure en ligne (100) comprend un capteur (9) connecté à la cuve (2) pour détecter une réflexion totale atténuée par spectroscopie moyen infrarouge du contenu de cuve (3) pour obtenir la première valeur d'extrait (A).

13. Appareil (1) pour la fermentation de la bière, comprenant une cuve (2) pour contenir un contenu de cuve (3) comprenant un mélange d'au moins de moût et de levure au cours d'un processus de fermentation, un dispositif de mesure en ligne (100) incluant un capteur (9) connecté à la cuve (2) pour obtenir une mesure d'une première valeur d'extrait (A) qui est représentative d'un niveau d'extrait du contenu de cuve (3), un dispositif de mélange (6, 7) configuré pour retirer le contenu de la cuve (2) et le réinjecter dans la cuve (2) pour mélanger le contenu de cuve (3), le dispositif de mesure en ligne (100) comprenant un dispositif de commande (10) pour commander automatiquement au moins le dispositif de mélange (6, 7) en fonction d'au moins la première valeur d'extrait (A).

14. Appareil selon la revendication 13, dans lequel le capteur (9) est connecté à la cuve (2) pour détecter la réflexion totale atténuée par spectroscopie en infrarouge moyen du contenu de cuve (3) pour obtenir la première valeur d'extrait (A) du contenu de cuve.

15. Appareil selon la revendication 13 ou 14, dans lequel le dispositif de commande (10) comprend une unité de traitement de données (101), et une mémoire de données non transitoires (102) contenant des données de commande pour exécution par l'unité de traitement (101), moyennant quoi le dispositif de commande (10) est configuré pour commander l'appareil pour qu'il réalise l'une quelconque des étapes énoncées dans les revendications 1 à 12 du procédé précédent.
